# EUROPEAN PATENT SPECIFICATION

(11) **EP 0 314 937 B1**
(45) Date of publication and mention of the grant of the patent: **13.12.1995**
(21) Application number: 88116645.8
(22) Date of filing: 07.10.1988
(51) Int. Cl.: A61B 5/14, A61N 1/365

(54) **Implantable blood oxygen sensor and method of use**
Implantierbarer Blutsauerstoffsensor und Verfahren zu seinem Gebrauch
Capteur implantable du taux d'oxygène dans le sang et procédé d'emploi

(30) Priority: 08.10.1987 US 107062
(43) Date of publication of application: 10.05.1989
(73) Proprietor: Pacesetter AB, 171 95 Solna (SE)
(72) Inventor: Barcel, James E., Simi Valley, CA 93065 (US); Hooven, Michael D., Valencia, CA 91355 (US); Cohen, Donald M., Encino, CA 91316 (US)
(74) Representative: Rees, David Christopher

(56) References cited:
- EP-A- 0 257 954
- US-A- 4 399 820

## Description

### BACKGROUND OF THE INVENTION

The present invention relates to implantable medical devices, and more particularly to an implantable medical sensor that can be used to determine the oxygen content of blood. In a preferred embodiment, the sensor is housed in a miniature hermetically-sealed capsule that is embedded into an implantable pacemaker lead of a rate-responsive pacemaker.

It is known in the art to use an implantable sensor to determine the oxygen content of blood. It is also known in the art to utilize such a sensor in combination with a pacemaker in order to adjust the pacing interval or frequency of the pacemaker as a function of the oxygen content of the blood. See U.S. Patents 4,202,339 and 4,399,820. Further, as described in U.S. Patent 4,399,820 (hereafter the '820 Patent), the prior art recognizes the advantages of incorporating an oxygen sensor made from a light-emitting diode (LED) and a light receiving transistor, or phototransistor (PT), into an implantable cardiac pacing lead.

Unfortunately, the pacing system, including the lead and sensor, described in the '820 Patent, while representing a significant advance in the art at the time of its development, still exhibits some problems which have heretofore hindered a widespread clinical use of such a pacing system. For example, while the LED and PT elements used to realize the oxygen sensor of the pacing system taught in the '820 patent are advantageously embedded into the pacing lead, they are done so in a way that does not guarantee a true hermetic seal, there being at least one annular insulation layer 35 through which body fluids could seep into the otherwise closed area where the LED element 32 and PT element 37 are located. Further, given the relatively large size of the annular elements used to create the pocket within which the LED and PT are located, e.g., the glass ring 39, and metal annular elements 31 or 31' and 34 or 34', the glass ring having a diameter approximately the same as the lead diameter, and given that the annular metallic elements have an outside diameter that must be firmly welded to the glass ring all around the circumference thereof, there is a relatively large weld that must not develop any leaks. This is not an easy task using conventional bonding techniques, especially given the periodic forces that are regularly placed on the lead as it moves or flexes within the heart or body.

Thus, disadvantageously the sensor taught in the '820 Patent is not easily sealed and protected from body fluids that might find their way into the lead, and even if it is initially sealed, it may not remain so with use. Needless to say, the presence of such body fluids within the lead could dramatically alter the optical properties and performance of the sensor system, as well as, the pacer output capabilities. Further, the arrangement shown in the '820 patent is very expensive, both in manufacturing time and cost. What is needed, therefore, is a more economical sealed sensor that can more readily be embedded into a pacing lead and that will remain tightly sealed throughout the life of the lead.

EP-A-0 257 954, which is an earlier application pursuant to art 54 (3) EPC, describes an oxygen sensor used with an implantable pacemaker. The sensor is formed as a hermetically sealed part of an electrode lead and implanted into a heart for measuring the oxygen saturation of the blood. The sensor comprises an infrared LED (IR-LED), a red LED and a phototransistor. The LEDs are activated in sequence emitting light through a transparent wall which light is reflected by the blood and detected by the phototransistor. The signals obtained by the phototransistor for each wavelength (IR and red) are stored in respective sample and hold circuits before the quotient IR/red is formed for determining the oxygen saturation.

### SUMMARY OF THE INVENTION

The present invention provides a miniaturized implantable medical sensor that optically senses the oxygen content of a body fluid to which the sensor is exposed, such as blood. In its completed form, the sensor is only about 7,62 mm (0.3 inches) long and 2,54 mm (0.1 inches) in diameter. Advantageously, the sensor includes an economical hybrid circuit, comprising a light-emitting diode (LED), at least one phototransistor, and one resistor, all mounted on a hybrid substrate which is in turn mounted on a titanium sensor body, the body and components all being inserted into a cylindrical sleeve. The sleeve is an assembly made of a length of glass tubing, comprised of soda lime glass with metal rings fused to each end. The ends of the cylindrical assembly are sealed to the titanium body through the use of titanium end rings. The titanium rings are permanently bonded to the walls of the glass tubing by applying heat at a specified temperature, which heat causes a permanent chemical bond to form between the titanium and the glass. Platinum feedthrough terminals pass through feedthrough holes in the sensor body and provide a means for making electrical contact with the hybrid circuit. These feedthrough holes are sealed with a high alumina (a type of ceramic) frit. The sealed sensor is embedded into a pacing lead and positioned near the distal end of such lead so that when the lead is implanted within a patient's heart, the sensor also resides in, or is near, the heart. Electrical contact is made with the sensor by connecting its feedthrough terminals to appropriate electrical conductors within the lead, which may be the conductors used for pacing/sensing of the heart.

In operation, the LED of the sensor is, in a preferred embodiment, energized with a stair-stepped current pulse. Energizing the LED with two known current levels in this fashion allows for meaningful analysis of the voltage-current relationship of the particular sensor used regardless of individual variations that exist between the components of the sensor, the lead conductors, or the ambient temperature.

When used with a pacemaker or similar stimulating device, the present invention thus provides a body-implantable sensor and lead that includes an implantable stimulating lead having a connector at one end thereof and electrode means at the other end thereof, the electrode means comprising means for electrically contacting body tissue when the lead is implanted in a body, and the connector comprising a means for interfacing the lead, both electrically and physically, with the desired stimulating device; a first insulated conductor having a distal end coupled to the electrode means and a proximal end coupled to the connector; sensor means forming an integral part of the lead for quantitatively sensing a specified characteristic of a body fluid proximal the sensor means, the sensor means including means responsive to a drive signal for generating an output signal that varies as a function of the specified body fluid characteristic; and means for transmitting the drive signal and the output signal between the sensor means and the connector.

The present invention also includes a method of using a medical sensor that has light-emitting diode means for emitting a light pulse that is directed to a desired type of organic material to be analyzed, which light pulse is then received by phototransistor means, a desired property of the organic material being determinable in accordance with the manner in which at least one detectable property of the light pulse is affected by the organic material; the method comprising the steps of: (a) exciting the light-emitting diode means with a first drive current for a first period of time; (b) exciting the light-emitting diode means with a second drive current for a second period of time immediately subsequent the first period of time; (c) monitoring the light pulses received by the phototransistor means during the first and second periods of time to determine the change in the light pulses as a result of having come in contact with the organic material; and (d) processing the amount of change identified in step (c) to determine a desired property of the organic material.

### BRIEF DESCRIPTION OF THE DRAWINGS

The above and other advantages and features of the present invention will be more apparent from the following more particular description thereof, presented in conjunction with the following drawings, wherein:
Fig. 1 is a schematic diagram of an oxygen sensor of the prior art;
Fig. 2 is a schematic diagram of an oxygen sensor in accordance with the present invention;
Fig. 3 is a representative family of curves showing the voltage-current relationship of the sensor of Fig. 2;
Fig. 4 is block diagram illustrating the manner in which the oxygen sensor of Fig. 2 is used to sense the reflective properties of blood, which reflective properties vary as a function of the oxygen content of the blood;
Fig. 5 is a block diagram showing the sensor of Fig. 2 inserted into the pacing lead of a rate-responsive pacing system;
Fig. 6A is a functional schematic diagram of a circuit configuration that is used to realize the sensor drive circuit of Fig. 4;
Fig. 6B is a functional schematic diagram of a circuit configuration that is used to realize the sensor process circuit of Fig. 4;
Fig. 6C is a timing diagram showing the interrelationship between various key signals used by the system of Fig. 5 and the circuits of Figs. 6A and 6B;
Fig. 7A is a perspective view of a preferred sensor body;
Fig. 7B is a sectional view of the sensor body of Fig. 7A;
Fig. 7C is an end view of the sensor body of Fig. 7A;
Fig. 8A is a top view of a hybrid version of the sensor circuit of Fig. 2 realized on a ceramic substrate (for clarity, reflector 150 is omitted form this view);
Fig. 8B is a side view of the hybrid circuit of Fig. 8A;
Fig 9A is a side view of a sensor body assembly, and shows the hybrid circuit of Fig. 8 mounted to the sensor body of Fig. 7, and shows feedthrough pins passing through the sensor body for the purpose of making electrical contact with the hybrid circuit;
Fig. 9B is an end view of the sensor body assembly of Fig. 9A;
Fig. 10A is an exploded side section view of a sensor lens assembly;
Fig. 10B is a side view of the assembled lens assembly of Fig. 10A;
Fig. 11 is a view of a reflector element before forming that may be inserted around the LED of the hybrid circuit;
Fig. 12 is a side view of the complete sensor assembly, including body, hybrid circuit, feedthroughs, lens, and reflector;
Fig. 13A is a side view of the sensor assembly of Fig. 12 embedded into a bilumen pacing lead;
Fig. 13B is a cross-sectional view of the lead of Fig. 13A taken along the lines 13B-13B;
Fig. 14 is a schematic representation of the lead of Fig. 13A;
Fig. 15A is a cross-sectional view of one embodiment of a bipolar pacing lead wherein the sensor assembly of Fig. 12 is embedded;
Fig. 15B is a schematic representation of the lead of Fig. 15A;
Fig. 16A is a cross-sectional view of another embodiment of a bipolar pacing lead wherein the sensor assembly of Fig. 12 is embedded;
Fig. 16B is a schematic representation of the lead of Fig. 16A;
Fig. 17 is a simplified schematic representation of a pacemaker and a bipolar pacing lead into which the sensor assembly of the present invention is embedded;
Fig. 18 is an alternative embodiment of a pacemaker and a bipolar pacing lead into which the sensor assembly of the present invention is embedded; and
Fig. 19 illustrates a process assembly tree for the sensor assembly.

### DETAILED DESCRIPTION OF THE INVENTION

The following description is of the best presently contemplated mode of practicing the invention.

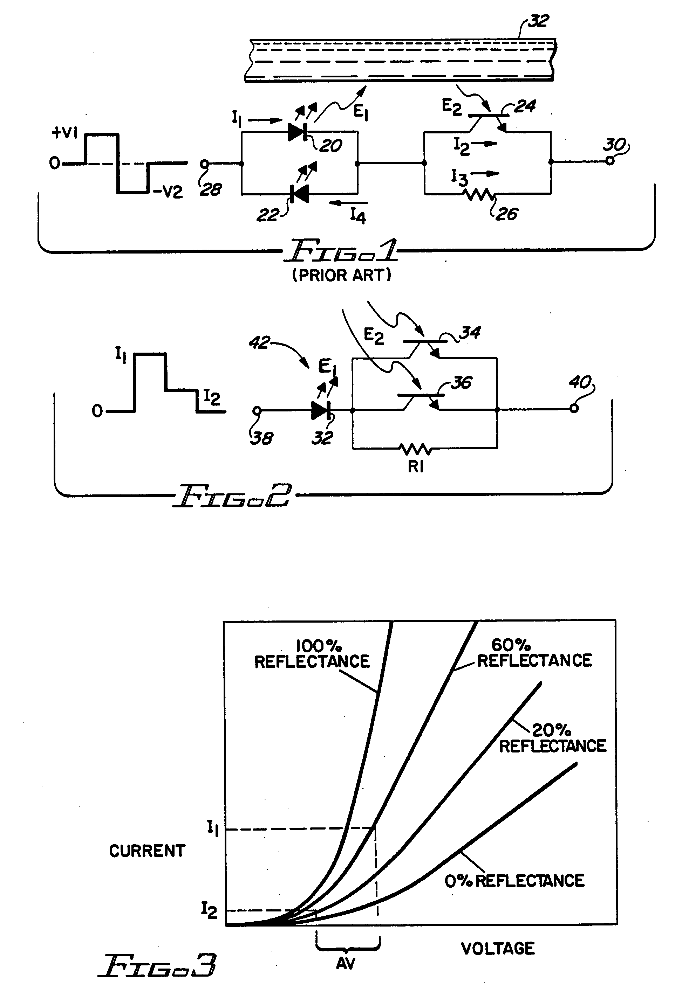

The invention will be described with reference to the drawings, wherein like numerals (or equivalent reference designators) are used to designate like parts throughout the following description. In order to better understand the present invention, it will help to first understand the operation of an oxygen sensor of the type that is known in the art. Accordingly, reference is first made to Fig. 1 wherein the schematic diagram of an oxygen sensor of the prior art is illustrated. The sensor includes two light-emitting diodes 20 and 22 connected in parallel, with the anode of diode 20 being connected to the cathode of diode 22, and the anode of diode 22 being connected to the cathode of diode 20. A phototransistor 24 is connected in parallel with a resistor 26, and the collector of the phototransistor 24 is connected to the same node as is the anode of diode 22 and the cathode of diode 20. The node comprising the anode of diode 20 and the cathode of diode 22 comprises one input terminal 28, and the emitter of phototransistor 24 and one side of the resistor 26 comprises another terminal of the sensor 30.

In operation, a bi-phase voltage pulse is applied across terminals 28 and 30. This bi-phase voltage pulse is also illustrated in Fig. 1 and includes a positive portion, having an amplitude of +V1; followed by a negative portion, having a negative amplitude of -V2. The positive portion of the bi-phase voltage pulse causes a current I₁ to flow through light-emitting diode 20, thereby causing light energy E₁ to be emitted by the LED 20. The light E₁ comes in contact with a desired body fluid 32, such as blood. Depending upon the properties of the fluid 32, a portion of the light energy E₁ is reflected back to the phototransistor 24. In Fig. 1, as well as in the other figures, that portion of light energy reflected back to the phototransistor is identified as E₂. Thus, in Fig. 1, the amount of current I₂ that flows through phototransistor 24 is a function of the light energy E₂ that is incident upon the base of the phototransistor 24. The balance of the current I₁ that does not flow through phototransistor 24, therefore, flows through the resistor 26. This current is identified as I₃. Thus, it is seen that I₁ is equal to I₂ plus I₃. The current I₂ varies as a function of the light energy E₂, thereby also affecting the amount of current I₃ that flows through resistor 26. The voltage developed across terminals 28 and 30 (which voltage is a function of the forward voltage drop across LED 20 and the voltage drop across resistor 26 caused by the current flow I₃) will thus vary as a function of the reflected light energy E₂ that is incident upon the phototransistor 24. Hence, by monitoring the voltage across the terminals 28 and 30, it is possible to get an indication of the reflectance properties of the fluid 32.

In order to determine the amount of voltage variation across terminals 28 and 30 caused by the current I₂, it is necessary to isolate other variations in this voltage from the measurement. This is typically done by causing current I₄ to flow through resistor 26 and LED 22 during the negative portion of the bi-phase voltage waveform. During this portion of the wave form, both the phototransistor 24 and LED 20 are back biased, and therefore no current flows through either of these devices. The value of I₄ is selected to be close to the value of I₁ so that the forward voltage drop across LED 20 will be approximately the same as the forward drop across LED 22. As can be appreciated by those skilled in the art, this technique works only if LED 20 and LED 22 are closely matched. Further, the method assumes that negligible leakage currents flow through phototransistor 24 and LED 20 when such are back biased. The technique is further complicated by the fact that a bi-phase voltage wave form must be generated, which typically requires more than one voltage source, or a complicated biasing scheme. Further, the configuration of Fig. 1 employs just one phototransistor 24 to receive the reflected light energy E₂. The sensitivity of the circuit to changes in the reflected properties of the fluid 32 would be vastly improved if additional phototransistors could be employed. Unfortunately, in order to keep the physical size of such sensors very small, such devices cannot use more than about four elements as shown in Fig. 1: two LED'S, one phototransistor, and one resistor.

In order to overcome these problems, the present invention provides a sensor 42 as shown in Fig. 2. Sensor 42 includes a single LED 32 connected in series with a parallel network made up of a first phototransistor 34, a second phototransistor 36, and a resistor R1. Advantageously, the configuration shown in Fig. 2, like that of Fig. 1, is limited to four elements, and therefore the size can be made very small. Further, the configuration of Fig. 2 uses only one LED 32, which LED 32 need not be matched with any other LED. A significant advantage of the circuit of Fig. 2 is that two phototransistors are employed rather than one. Each phototransistor is capable of receiving the reflected light energy, E₂, thereby significantly improving the sensitivity of the device. Another advantage of the circuit configuration of Fig. 2 is that only one voltage source is required, the LED 32 being driven by a stair-stepped current pulse. A first portion of the current pulse provides a drive current I₁ that causes LED 32 to emit light energy E₁. The second portion of the stair-stepped current pulse provides a current I₂, which is chosen to effectively be on the knee of the voltage-current characteristic transfer curve of the LED 32. That is, the current I₂, is selected at a low value at which very little, if any, light energy E₁ is emitted by the diode E₂.

At either current level, the voltage developed and measured thus provides an indication of impedance at that current level. Hence, a differential measurement may be made between the voltage (or other parameter) developed at current value I₁ and at current value I₂, which differential measurement can be used in a variety of ways, such as is discussed in connection with Fig 3.

Fig. 3 depicts a family of curves showing the voltage-current relationship of the sensor of Fig. 2. The change in voltage developed across terminals 38 and 40 of sensor 42 of Fig. 2 provides an accurate measurement of the amount of reflected light energy received by photo-transistors 34 and 36. Hence, by monitoring this change in voltage, the sensor 42 of Fig. 2 is able to provide an indication as to the reflective properties of the fluid 32 to which the light energy E₁ is exposed. These reflective properties, in turn, can be correlated to a desired property of the fluid 32, such as the oxygen content of the fluid. This correlation is known and documented in the art.

Referring next to Fig. 4, a block diagram is shown illustrating the manner in which the oxygen sensor 42 of Fig. 2 is used to sense the reflective properties of blood. The sensor of Fig. 2, identified in Fig. 4 as the sensor 42, is positioned within an area of a living body where blood 44 is able to come in contact with the light energy E₁ emitted by the sensor. Typically, the sensor 42 will be placed within a vein that is carrying blood back to the lungs, or within the heart itself. A sensor drive circuit 46 provides the stair-stepped current pulse needed to drive the sensor 42. Similarly, a sensor process circuit 48 monitors the voltage developed across the sensor terminals 38 and 40. Appropriate timing signals 50 are shared between the sensor drive circuit 46 and the sensor process circuit 48. Further, in order to synchronize the sensing function of the sensor 42 with other events, the sensor drive circuit 46 and the sensor process circuit 48 typically receive a clock signal 52 and a timing reference signal 54 from a location external to these circuits. For example, when the sensor 42 is used with an implanted cardiac pacemaker, the clock signal 52 is obtained from the circuits within the pacemaker. Similarly, the reference signal 54 is typically a signal indicating a cardiac event, such as a V-pulse or R-wave signal, which signals indicate that the ventricle of the heart has either been paced or that a ventricular contraction has been sensed.

The use of the sensor 42 with an implanted rate-responsive pacemaker 56 is further illustrated in Fig. 5. In Fig. 5, it is seen that the drive circuit 46 and the sensor circuit 48 are included within a pacemaker housing, which housing is made to be implantable in a human body. Included within the rate-responsive pacemaker 56 are conventional pacemaker circuits 58. The drive circuit 46 and the sensor circuit 48 are coupled to the pacemaker circuits 58 in the manner above-described. That is, the clock signal 52, as well as a V/R signal (signifying either an R-wave has been sensed or a V-stimulation pulse has been generated) are provided from the pacemaker circuits 58 to the drive circuit 46 and the sensor circuit 48. A pacing lead 60, connected to the pacemaker housing 56 by way of a conventional bipolar pacer connector 62, allows the pacemaker to deliver stimulation pulses to a heart 64 at a distal electrode tip 66 through conductor 70. This same conductor 70 allows the pacemaker circuits to sense cardiac events occurring near the lead tip 66. The sensor 42 is advantageously embedded within the pacemaker lead 60 at a location near the distal tip so as to place the sensor 42 within the heart 64. Further, when positioned properly within the heart, the lead is curved in a manner that causes the sensor to face blood just prior to the blood's passage through the heart's tricuspid valve. The terminal 38 of the sensor 42 is connected to a separate conductor 68 of the lead 60. The other terminal 40 of the sensor 42 is connected within the lead to the conductor 70. The sensor process circuit 48 develops a control signal 49 that is representative of the reflectance properties of the blood (and hence relatable to the amount of oxygen that has been sensed within the blood). This control signal 49 is presented to the pacemaker circuits 58 and is used as a physiological parameter to control the rate at which the pacemaker circuits deliver a stimulation pulse to the heart. Thus, the configuration shown in Fig. 5 is representative of a rate-responsive pacemaker wherein the rate of the pacemaker varies as a function of the sensed oxygen content of the blood that comes in contact with the sensor 42.

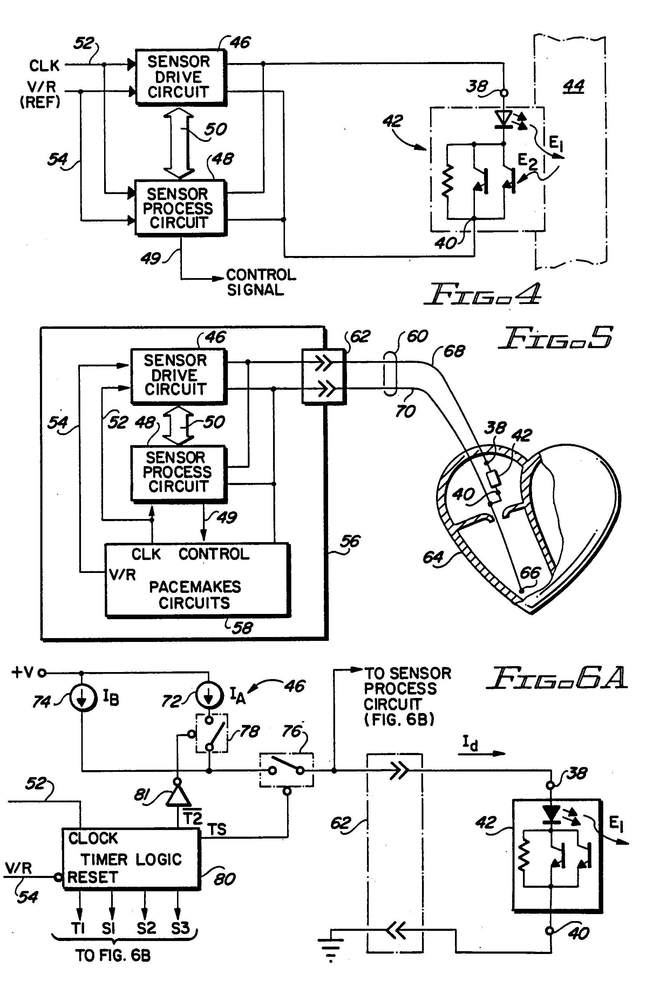

Referring next to Figs. 6A, 6B, and 6C, functional schematic diagrams of the sensor drive circuit 46 (Fig. 6A) and the sensor process circuit 48 (Fig. 6B) are shown. Also included, in Fig. 6C, is a timing diagram showing the interrelationship between various key signals used by the circuits of Figs. 6A and 6B. In Fig. 6A, it is seen that the sensor drive circuit 46 comprises a first current source 72 (that provides a first current I_{A}) and a second current source 74 (that provides a second current I_{B}). The current source 74 is connected to the terminal 38 of the sensor 42 by means of a first switch 76. The current source 72 is switchably connected in parallel with the current source 74 by means of another switch 78. The switches 76 and 78 are controlled by logic signals generated by timer logic 80. As depicted in Fig. 6A, a high logic signal applied to the control input of switches 76 and 78 causes the switches to close. The timer logic 80 generates appropriate timing signals T1, T2, TS, S1, S2 and S3, having a relationship as shown in Fig. 6C. A clock signal 52, and a reset signal 54 (which may be the V/R signal from the pacemaker) are also provided as input signals to the timer logic 80. Conventional circuitry within timer logic 80, known to those skilled in the art, is used to generate the signal patterns shown in Fig. 6C.

As depicted in Fig. 6C, the V/R signal 54 resets the timer logic 80. As previously indicated, the V/R signal indicates that the ventricle of the heart has been paced with a V stimulation pulse, or that an R-wave has been sensed, indicating a natural contraction of the ventricle. In either event, there is a certain time period after such event during which the heart is refractory and the pacemaker circuits are inoperative. It is during this time period that the sensor 42 can be pulsed in order to measure the oxygen content of the blood. Thus, at a desired time T_{W} after the V/R signal, a sample pulse T_{S} is generated. This sample pulse T_{S} may be further divided into a first portion t₁ and a second portion t₂. During the sample pulse T_{S}, the switch 76 is closed, thereby allowing the current I_{B} from current source 74 to flow through the sensor 42. Further, during all but the time period T₂, the switch 78 is closed, thereby allowing the current I_{A} from current source 72 to also flow to the sensor 42 when switch 76 is closed. The net effect of this action is to have both currents I_{A} plus I_{B} flow through the sensor 42 during time t₁, and having only current I_{B} flow through the sensor 42 during time t₂. Thus, the desired stair-step current pulse is provided to the sensor 42. Typically, the energizing of the sensor 42 will occur some 10-20 milliseconds after the V/R signal (that is, T_{W} is approximately 10-20 milliseconds), however, it is to be understood that other times could be used. The width of t₁ and t₂ is on the order of 120 microseconds, thereby providing a total sensor measurement time of 240 microseconds. The stair-stepped current provided to the sensor 42 is represented in Fig. 6 as the drive current I_{d}. Typically, I_{B} has a value of about 0.3 ma, and I_{A} has a value of about 0.9 ma (making the maximum drive current equal to about 2.0 ma).

Referring next to Fig. 6B, the functional schematic diagram of the sensor process circuit 48 is depicted. It is the function of this circuit to measure the voltage developed across the sensor 42 during each portion t₁ and t₂ of the time T_{S} during which the sensor 42 is active. This is accomplished through the use of two sample pulses S1 and S2. As seen in Fig. 6C, the sample pulse S1 occurs during the latter portion of the time t₁ of signal T1, and the sample pulse S2 occurs during the latter portion of the time t₂ of the signal T2. The sampling is done towards the latter portion of each of these sample times in order to allow the voltage developed across the sensor 42, V_{S}, to settle down before the measurement is made.

Referring again to Fig. 6B, it is seen that the sensor process circuit 48 connects the terminal 38 of the sensor 42 to amplifiers 86 and 88 through switches 82 and 84 respectively. Switch 82 is closed during time T1, and switch 84 is closed during time T2. The outputs of amplifiers 86 and 88 are fed into the inputs of sample and hold circuits 90 and 92, respectively. Sample signal S1 controls the operation of the sample and hold circuit 90; while the sample signal S2 controls the operation of the sample and hold circuit 92. Both sample and hold circuits 90 and 92, as well as the amplifiers 86 and 88, and the switches 82 and 84 (and the switches 76 and 78) are of conventional design. It is the function of the sample and hold circuits 90 and 92 to present and hold at the output terminal the voltage appearing at the input terminal during the sample time.

The output of the sample and hold circuits 90 and 92 is directed to a conventional differential amplifier or summing circuit 94. During sample time S3, which is subsequent the energization of the sensor 42 by a few milliseconds, the differential amplifier 94 has as inputs two voltages held by the respective sample and hold circuits 90 and 92 in order to determine the difference therebetween. This difference in voltage corresponds to the difference in voltage represented in the family of curves of Fig. 3, and is therefore relatable to a particular reflectance value. Hence, the conversion circuit 96, uses this difference voltage in order to generate the control signal 49 that provides a quantitative measure of the reflectance properties measured. Advantageously, this control signal can be presented to the rate-responsive pacemaker circuits in order to control the pacing interval thereof, as shown in Fig. 5.

In a preferred embodiment, the rate-responsive pacemaker circuits 58 (Fig. 5) include a microprocessor. Hence, the conversion circuit 96 is implemented using appropriate analog-to-digital conversion techniques and a subroutine within the microprocessor that converts the digitized difference voltage presented thereto to a particular reflectance amount. In turn, this reflectance amount is converted to an appropriate control voltage that is used to adjust the pacing interval of the pacemaker circuits, using conventional rate-responsive pacemaker control techniques.

It is noted, with reference to Fig. 6B, that the gain of amplifiers 86 and 88 can be selected as desired in order to optimize the performance of the sensor processor circuit 48. The gain of each amplifier is preferably a programmable parameter that can be adjusted, using conventional programming techniques, in order to achieve a desired result. Reference is made to U.S. Patent 4,232,679 for teaching the fundamentals of programming various parameters in order to alter the operation or performance of an implanted device, including changing the gain of an amplifier.

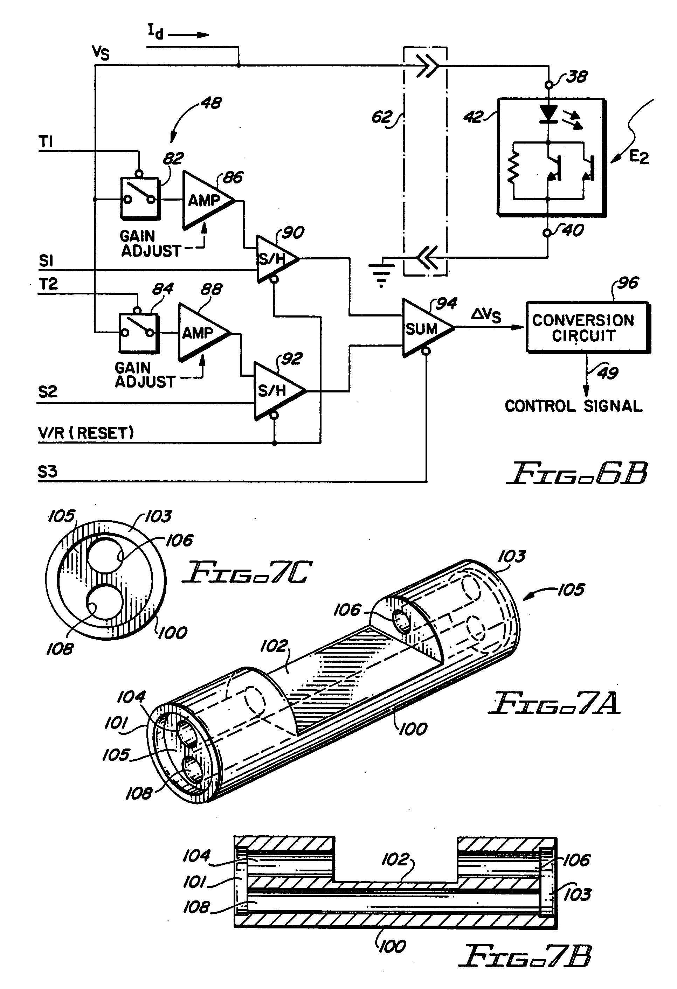

Referring next to Figs. 7A, 7B, and 7C, there is shown a perspective, sectional view and end view, respectively, of a sensor body 100. As indicated in Fig. 7A, the sensor body 100 comprises a cylindrical shaped member, a center portion of which has been removed so as to provide a flat portion 102 upon which the sensor circuitry, as described below, may be mounted. Circumferential ridges 101, 103 at each end of the body 100 define recessed end portions 105. These ridges advantageously provide a uniform thickness welding surface to which titanium rings 144 and 146 can be welded, as described below. A hole 104 passes through one end of the sensor body 100 to the platform area 102; similarly, another hole 106 passes through the other end of the sensor body 100 to the platform area 102. A longer hole 108, passes through the entire length of the sensor body 100 under the platform area 102.

In the preferred embodiment, the sensor body is made from titanium. It has a length of approximately 0.3 inches, and a diameter of approximately 0.1 inches.

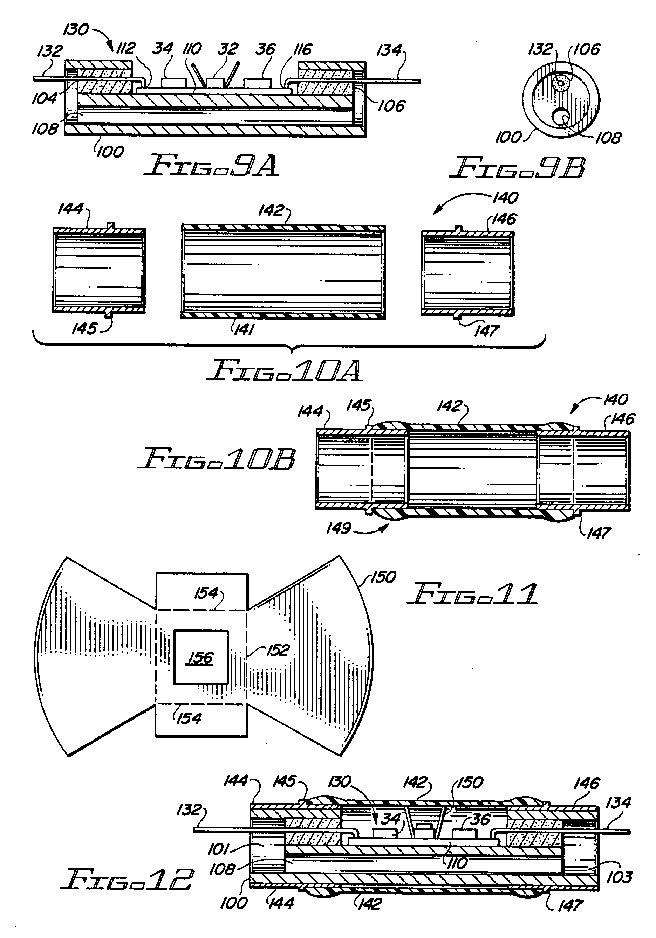

The sensor circuitry is mounted on a ceramic substrate 110, as shown in Figs. 8A and 8B. Fig. 8A shows a top view of the ceramic substrate and Fig. 8B shows a side view thereof. The ceramic substrate 110 has conductive regions (a metallic layer) deposited and etched thereon using conventional techniques. As shown in Fig. 8A, there are three conductive areas or regions (distinguished by a different style of cross-hatching), each electrically insulated from the others, that are placed on the substrate 110. A first conductive region connects one end 112 of the substrate with a center area 114 by way of a conductive track 113. A second conductive region connects the other end of the substrate 116 to a front edge of the substrate 118. Finally, a third conductive region connects a first pad area 120 with a second pad area 122 by means of a conductive track 123. The anode of LED 32 (see Fig. 2) is bonded to the central pad or conductive area 114 using a conductive polymer, in conventional manner. (A conductive reflector 150, shown in Figs. 11 and 12 may be first bonded to area 114, with the cathode of LED 32 being bonded to the reflector 150. However, for clarity, this reflector 150 is omitted from Fig. 8A.) Similarly, the collector of phototransistor 34 is bonded to pad 120, and the collector of phototransistor 36 is bonded to pad 122. This bonding is done using a commercially available bonding methods, such as eutectic bonding techniques. A thin film resistor of an appropriate value is deposited or otherwise placed on the ceramic substrate 110 in conventional manner so as to be electrically connected between pad area 114 and the end portion of the substrate 116. In the preferred embodiment, this resistor R1 has a value of approximately 500 ohms. A first bonding wire 124 is connected between the emitter of phototransistor 34 and the front edge conductive area 118. Similarly, another bonding wire 126 connects the emitter of phototransistor 36 and the area 118. Finally, still another bonding wire 128 connects the cathode of LED 32 to the conductive track 123 that connects the pad 120 to the pad 122. Conventional hybrid circuit mounting and bonding techniques are used in order to bond the LED and phototransistors to their desired locations on the ceramic substrate 110, and in order to connect the bonding wires 124, 126 and 128 to their desired locations.

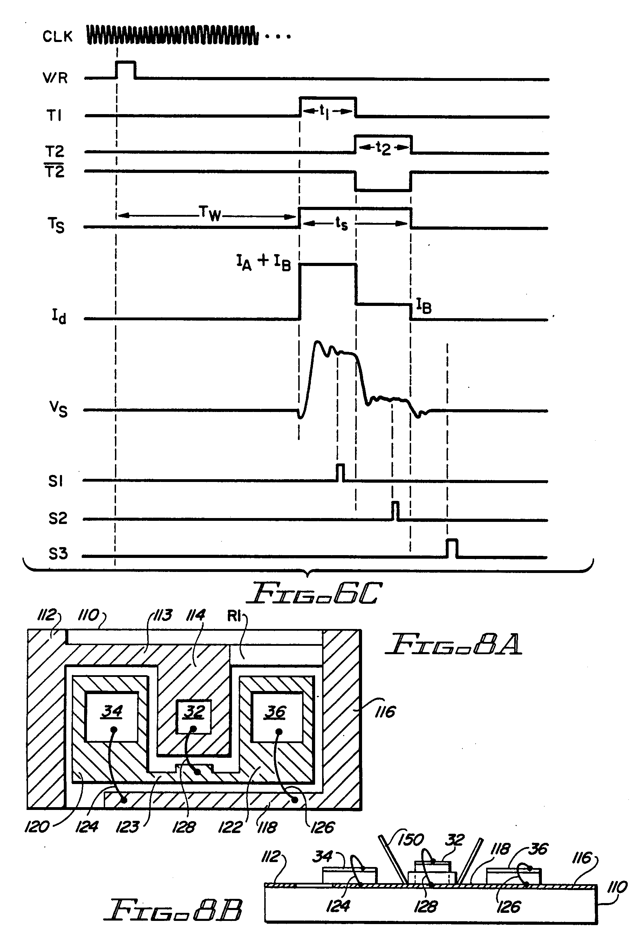

Referring back to Fig. 7, the short holes 104 and 106 of the sensor body 100 provide a means for allowing feedthrough wires 132 and 134 (Fig. 9) to pass from the sensor end 105 to the flat center portion 102. Feedthrough wires 132 and 134 are, in the preferred embodiment, made from 0.005 inch diameter 80/20 platinum-irridium (Pt-Ir) wire. It has been shown in the prior art that the Pt-Ir material is very stable, with a tendency to neither migrate nor oxidize. The contact that the Pt-Ir makes with the printed gold on the circuit is quite reliable. A high Al₂0₃ frit is preformed over respective lengths of such wire, and the wire-coated-with-frit is inserted into holes 104 and 106. The sensor body and frit are then fired at a temperature and duration compatible to form a hermetic, biocompatible, stable seal in holes 104 and 106 through which the feedthrough wires 132 and 134 pass.

After the sensor body 100 has the feedthrough wires 132 and 134 inserted and sealed into holes 104 and 106, it is combined with the hybrid assembly of Fig. 8 to form a sensor body assembly 130 as shown in Figs. 9A and 9B. The bottom side of the ceramic substrate 110 is bonded to the platform area 102 of the sensor body. The feedthrough wires 132 and 134 are then bent and welded to the end pads 112 and 116, respectively, of the ceramic substrate 110. The pins 132 and 134 thus provide a means for making electrical contact with the sensor circuitry located on the substrate 110. It is noted that the pin or feedthrough 132 electrically corresponds to the terminal 38 in Fig. 2, and the pin or feedthrough 134 electrically corresponds to the terminal 40 in Fig. 2.

With reference now to Fig. 10A, an exploded side sectional view of a sensor lens assembly 140 is shown. This assembly includes a glass tube 142 to which two end rings 144 and 146 will be attached. Fig. 10B shows the sensor lens assembly 140 in its assembled form. The end rings 144 and 146 are slideably inserted into desired locations at each end of the glass tube 142 until ridges 145 (on the outer diameter of end ring 144) and ridge 147 (on the outer diameter of end ring 146) abut up against the end of the glass tube 142. Heat is then applied to each end of the glass tube 142 in order to permanently bond the end rings 144 and 146 to the glass tube. This heat is applied by inductive heating of the metal rings 144 and 146. A temperature of around 700 degrees centigrade for 30-60 seconds is used for this purpose. Applying this amount of heat causes the glass tube to deform slightly as shown at 149 in Fig. 10B.

In the preferred embodiment, the glass tube 142 is soda lime glass. This type of material advantageously chemically reacts with the titanium end rings 144 and 146 when heat is applied as above described in order to form a very tight, chemical bond between the titanium end rings and the glass tube.

For the seal to be made reliably, several conditons must be satisfied. The temperature coefficients of expansion and contraction of the two materials must be matched to within a few parts per million per degree Celcius. The wall thicknesses, particularly of the metal rings, must be small (in the case of the metal rings, 0.002 inches). The annular clearance between the glass and metal must be uniform and small. The temperature to which the glass is heated must exceed the softening point without reaching the melting point. This temperature must be controlled such that there are not more than trivial, tensile residual stresses in the glass upon recovery to normal dimensions near room temperature.

Referring next to Fig. 11, there is shown a view of an unformed reflector 150 that is designed to be inserted around and/or under the LED 32 on the ceramic assembly 130. The reflector is machined by standard photo chemical etching means. The reflector may, in a subsequent operation, be formed into the appropriate shape by a tool and die. Large wings of the reflector 150 are folded along lines 152, and the short stubs of the reflector 150 are folded along the lines 154. The folded reflector assembly is, in the preferred embodiment, placed under the LED 32 during assembly. The reflector is made of a conductive material, such as brass or titanium. Once in place, the reflector will electrically connect the LED 32 to pad 114. Attachment is made with conductive adhesive, such as Ag epoxy or Polyimide. The reflector serves two purposes: (1) it increases the light launched into the blood that would otherwise not directly impinge upon the lense; and (2) it reduces the amount of light traveling directly from the LED to the phototransistor without first interacting with the blood.

Next, referring to Fig. 12, a sectional view of the completed sensor assembly 42 is shown. This assembly includes the sensor lens assembly 140 into which the hybrid sensor assembly 130 has been inserted. The titanium sensor body 100 is laser welded to the titanium end rings 144 and 146 using conventional laser welding techniques. The ends of the sensor body have been designed to simplify the laser welding process and to decrease the rejection ratio of this procedure. Flanges 101, 103 provide ends that form uniform, thin surfaces for welding to the metal rings of the lense assembly. The annular clearance between the rings and the body is kept to less than a few thousandths of an inch. This requires strict adherence to tolerances on the linearity of the lense assembly. The weld flanges offer the following advantages. They permit low laser energy to be used for the welding process since both metal pieces are thin. They allow a strong weld, since both the rings and the flanges are of similar thickness. They allow a uniform laser power density to be used since the flanges are of a constant wall thickness. Furthermore, they increase the separation between the high intensity laser beam and the susceptible components, e.g., feedthrough glass and electronics.

Once this weld has been completed, the hybrid sensor assembly 130 is totally sealed within the sensor assembly. Typically, the tightness or hermeticity of the seal is tested to at least 10⁻⁸ cc air/sec at one atmosphere pressure. However, even though thus sealed, the sensor circuit can readily function as above-described in connection with Figs. 2-6 by applying the appropriate drive signals or voltage measurement signals to the feedthrough pins 132 and 134. Advantageously, light emitted by the LED 32 is directed by the reflector 150 up through the soda lime glass sensor wall 142. Any reflections of this light can then similarly pass back through the lens 142 to the phototransistors 34 and 36. The sensor as shown in Fig. 12 can thus be incorporated within a desired measurement system and implanted within a patient for the purpose of sensing the reflective properties of a body fluid to which the sensor is exposed.

In the preferred embodiment, the sensor utilizes spectrophotometric analysis of the reflectance of light by blood (a body fluid). This principle has been described in the art, and it has been shown that reflectance is related to oxygen saturation. That is, oxygen saturation of whole blood can be estimated by analyzing the optical intensity of reflected light. For purposes of the present application, this means that the degree of light reflectance incident on the phototransistors 34 and 36 at a wavelength of 660 nm is a function of the mixed venous oxygen (MVO₂) concentration in the blood. A high MVO₂ concentration will reflect more light to the phototransistors, while a lower MVO₂ concentration will reflect less. It has been determined that roughly 18% reflectance occurs for the highest possible concentration of MVO₂ in the blood. This reflectance value varies to 0% as the MVO₂ concentration changes. (It is noted that in Fig. 3, the curve labeled 100% corresponds to the maximum reflectivity possible.) Furthermore, sensor signal amplitude is directly proportional to the intensity of the reflected light.

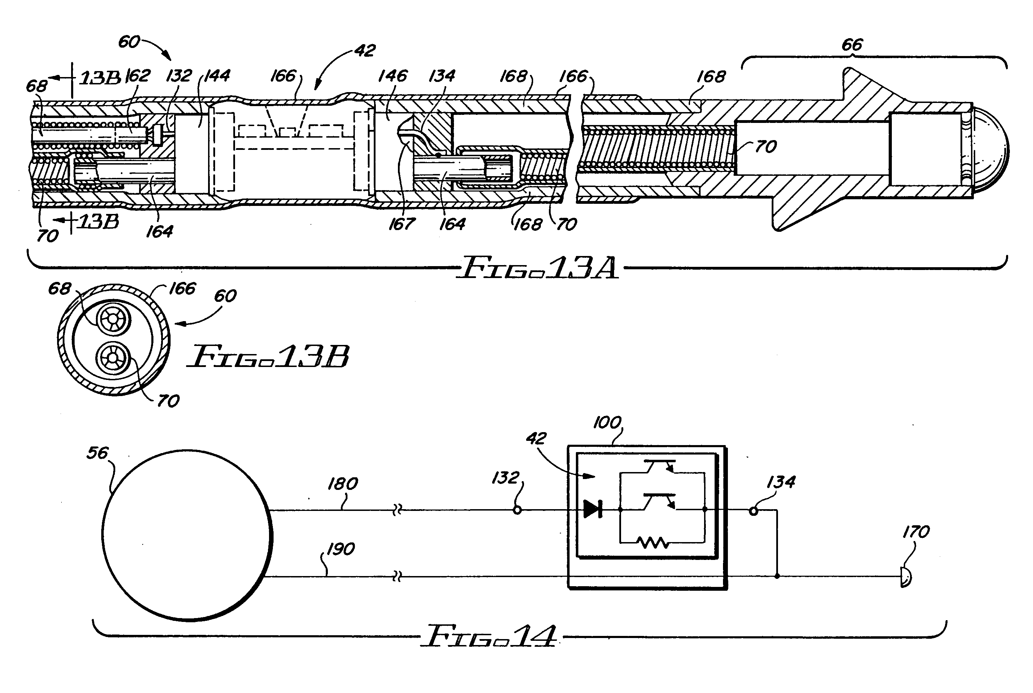

As has been indicated, in the preferred embodiment, the sensor 42 is embedded within a pacing lead 60 as shown in Fig. 13A. The pacing lead 60 is preferably a bilumen pacing lead having two conductors 68 and 70 passing through most of the length thereof. These conductors may be of conventional type, typically realized with a helically wound conductive wire. A cross-sectional view of the bilumen portion of the pacing lead 60 is shown in Fig. 13B

It is noted that while a bilumen pacing lead is shown in Figs. 13A and 13B, a coaxial type pacing lead could also be employed for the lead, and conventional break-out techniques could be used to separate the two coaxial conductors so that they could interface with the sensor as shown in Fig. 13A. In practice, such conventional break-out techniques are used towards the proximal end of the lead 60, not shown in Fig. 13A, in order to allow a conventional coaxial bipolar connector to interface with the pacemaker connector 62.

In Fig. 13A, the feedthrough pin 132 is connected to the conductor 68 using a conventional crimp connector 162. The materials selected for the crimp connector 162 and the conductor 68 should not create a galvanic potential that would promote corrosion. In the preferred embodiment, Pt-Ir and stainless steel provide a strong, non-corrosive bond. A stylet tube 164, made from stainless steel hypodermic tubing, coated with a thin polyimide coating over the portion of the tubing that may come in contact with the titanium sensor body 100, is passed through the lower hole 108 of the sensor. (Polyimide is a commercially available insulation material.) The purpose of the polyimide coating is to insulate the stylet tube 164 from the titanium sensor body 100. (Such insulation is optional, but is believed to be beneficial in order to prevent the sensor body 100 from acting as an electrode when a high current stimulation pulse passes through the lead conductor 70.) The helically wound conductor 70 is connected to the stylet tube 164 in a conventional manner at both ends of the stylet tube. In the preferred emdobiment, the stylet tube 164 is of the same material as the helically wound conductor 70. The feedthrough terminal 134 is bent down to make contact with the stylet tube 164 and is welded or bonded to the stylet tube using conventional laser welding or other bonding techniques. Epoxy fills the space between the protruding portions of the end rings 144 and 146, and further helps to seal the entire assembly.

Advantageously, a thin layer of polyurethane 166, or other suitable material, coats the length of the lead 60 along the length of the lead where the sensor is located. (This thin layer 166 could also coat much of the length of the lead, as is commonly done with many pacing leads.) This coating is in addition to the silicone rubber tubing 168 that forms the outer body of the lead. The polyurethane layer 166 is substantially transparent to the light energy emitted by the LED 32 (which light has a wavelength on the order of 660 nm). Advantageously, the polyurethane coating presents a transparent medium for light transmission and a benign coating which minimizes tissue growth, thereby keeping the "window" through which the light energy must pass, relatively clean.

The manner of making and assembling a pacemaker lead of the type shown in Fig. 13 is known in the art. What primarily distinguishes the lead 60 from conventional pacing leads is the use of the sensor 42, and the manner in which conductor 70 passes through the sensor body 100.

A schematic representation of the pacemaker lead of Fig. 13A is shown in Fig. 14 wherein a pacer 56 has a two-conductor lead attached thereto. A first conductor 180 attaches to the input terminal 132 of the sensor 42. A second conductor 190 passes through the sensor and is attached to a tip electrode 170, and to the output terminal 134 of sensor 42.

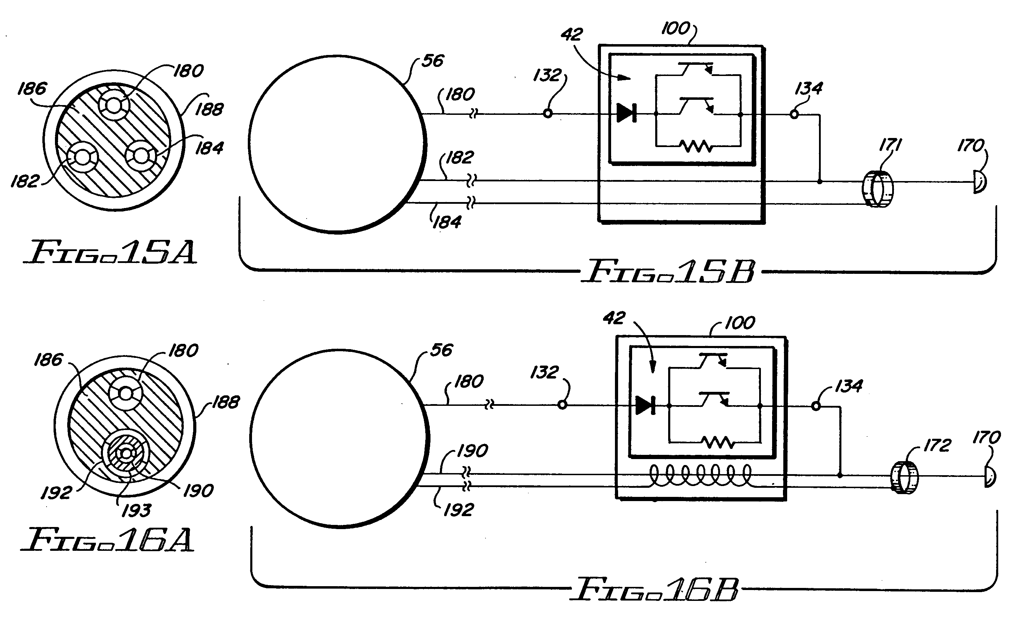

As an additional embodiment, it should be noted that the sensor 42 could also be incorporated into a bipolar pacing lead. If bipolar pacing is employed, two conductors need to pass by the sensor in order to make electrical contact with the tip and ring electrodes of the bipolar lead, respectively. If a separate additional conductor is used to contact the sensor, the body of the bipolar lead (assuming the sensor 42 is embedded near the distal tip of the lead) must have three conductors therein. One possible configuration to achieve this result is a tri-lumen lead of the type shown in the sectional view of Fig. 15A and the schematic representation of Fig. 15B, wherein a first conductor 180, a second conductor 182, and a third conductor 184 are encased in silicone rubber 186, which is coated with a thin layer of polyurethane 188. Thus, for the tri-lumen embodiment of Fig. 15, the titanium sensor body 100 (Fig. 7) has two long holes 108 passing through the lower half thereof.

Another possible configuration is a bilumen pacing lead as shown in the sectional view of Fig. 16A and corresponding schematic representation of Fig. 16B. Figs. 16A and 16B are similar to Figs. 15A and 15B, respectively, but the lower lumen of Fig. 16A contains coaxial conductors 190 and 192, with conductor 190 being of a smaller diameter helix and inside of the larger helix of conductor 192, each insulated from the other with conventional insulating material 193. In Fig. 16B, it is seen that the innermost conductor 190 of the coaxial conductors goes to the tip electrode 170 and to the sensor output 134. The outer conductor 192 goes to the ring electrode 172 of the bipolar lead.

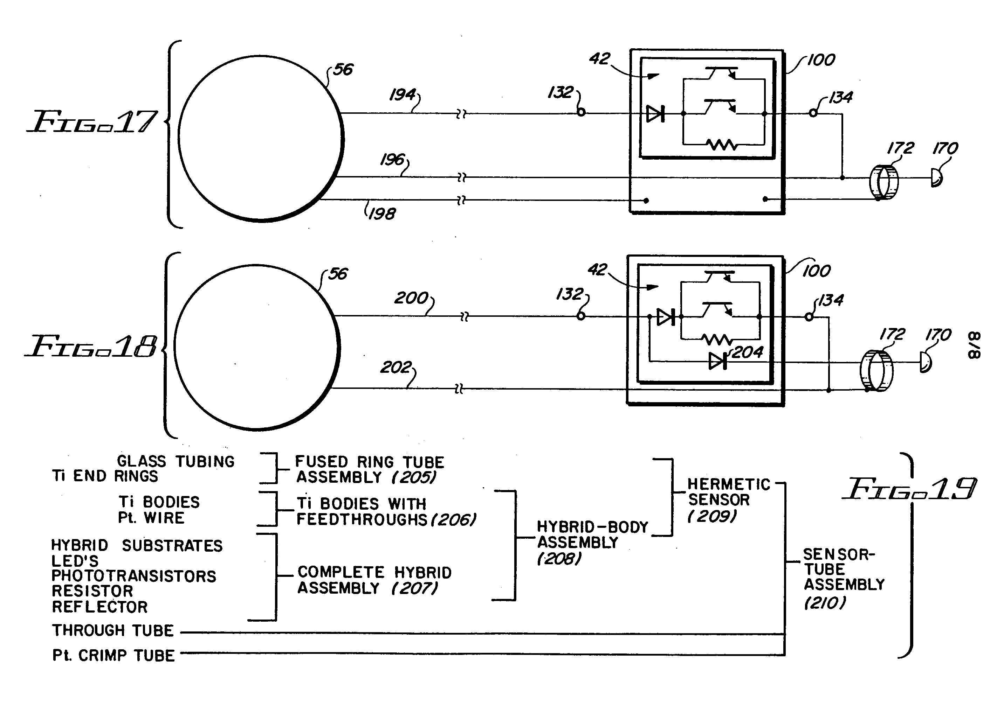

It should also be pointed out that the titanium sensor body 100 is itself an electrical conductor. As such, the body itself, or a contiguous portion of it, could function as the ring electrode in a bipolar mode. Alternatively, one of the conductive paths through the sensor assembly (to reach a distal tip or ring electrode, or to make contact with the return pin 134 of the sensor 42) could be the titanium body 100. In accordance with this approach, a conductor within the lead is securely attached to the proximal end of the sensor body 100, and a continuation of this conductor is attached to the distal end of the sensor body 100. This approach is schematically illustrated in Fig. 17, wherein a pacemaker 56 has a three-conductor lead attached thereto. A first conductor 194 attaches to the input terminal 132 of the sensor 42. A second conductor 196 passes through the sensor and is attached to a tip electrode 170, as well as the output terminal 134 of the sensor 42. (The attachment of the terminal 134 could be made internal to the sensor 42, if desired.) A third conductor 198 is electrically attached to the proximal end of the titanium body 100. The ring electrode 172 is attached to the distal end of the titanium body 100.

Advantageously, the configuration of Fig. 17 can be used to pace in either a unipolar or bipolar mode of operation. During unipolar operation, no signals or voltage potentials are applied within the pacer 56 to conductors 194 or 198. Rather, a negative pacing pulse is applied to conductor 196, and the positive voltage potential within the pacemaker 56 is applied to the can or case of the pacemaker 56 in conventional manner. Thus, the distal tip 170 paces unipolarly, cathodically. During operation of the sensor 42, the desired sensor drive signal is applied to conductor 194, and the negative voltage potential within the pacemaker 56 is applied to conductor 196. The can of the pacemaker 56 is allowed to float (not connected to any voltage potentials). Thus, current passes only through the sensor, not through the tip electrode 170.

During a bipolar pacing operation, no signals or voltage potentials are applied to conductor 194. A negative pacing pulse is applied to conductor 196 and the positive voltage within the pacemaker 56 is connected to conductor 198, which positive voltage is electrically connected to ring electrode 172 by way of the conductive titanium sensor body 100. This negative voltage also appears at the output terminal 134 of the sensor 42, but because the sensor 42 is floating (no signals connected to input terminal 132), this voltage does no harm. During operation of the sensor 42, the sensor drive signal is applied to conductor 194, and the negative voltage potential within the pacemaker 56 is applied to conductor 196. The can or case of the pacemaker 56 floats. Thus, current passes only through the sensor, not through the tip electrode 170.

Referring to Fig. 18, there is shown still another embodiment of the present invention used with a bipolar pacing lead configuration in which the pacing lead contains only two conductors 200 and 202. A blocking diode 204 is added to the sensor circuit. In accordance with this approach, both conductors of the lead are used during sensor operation: conductor 200 for the drive signal connected to the input terminal 132 of sensor 42 and conductor 202 for the return path connected to the output terminal 134 of sensor 42. Similarly, both conductors are used during conventional bipolar pacing/sensing: conductor 200 being connected to the tip electrode 170 through diode 204 and conductor 202 being connected to the ring electrode 172. During operation of the sensor 42, the sensor drive signal is applied to conductor 200, and the negative voltage is applied to conductor 202, which back biases the diode such that current passes only through the sensor, not through the tip electrode 170. Thus, both conductors within the lead are used for two functions. This embodiment assumes that the sensor 42 is not operational at a time when the tip/ring electrodes of the pacing lead are operating. Fortunately, as has been explained previously in connection with Figs. 5-6, a pacemaker is not operative during a refractory time period, and the sensor 42 could thus operate during this refractory time period.

As with the configuration of Fig. 17, the configuration of Fig. 18 can be used advantageously to pace in either a unipolar or a bipolar mode of operation. Unipolar pacing is achieved by floating conductor 202, applying a negative pacing pulse to conductor 200, and connecting the positive voltage potential of the pacemaker to the case or can of the pacemaker 56. Bipolar pacing is achieved by applying a negative pacing pulse to conductor 200, floating the pacemaker case, and connecting the positive voltage potential to conductor 202. Sensor operation is achieved by connecting the sensor drive signal to conductor 200, connecting the negative voltage potential of the pacemaker 56 to conductor 202, and floating the pacemaker case. These connections for each of these operations of the configuration shown in Fig. 18 are summarized in Table 1.

**TABLE 1**

| Conductor | Bipolar Pacing | Unipolar Pacing | O₂ Sensing |
|---|---|---|---|
| 200 | - | - | + |
| 202 | + | Float | - |
| Case/Can | Float | + | Float |

Representative switching circuitry needed within the pacemaker 56 in order to effectuate the various connections summarized in Table 1 may be carried out as detailed, for example, in pending patent application PACEMAKER HAVING PROGRAMMABLE CONFIGURATION, Serial No. 896,542, filed 8/13/86, assigned to same assignee as is this application (the '542 application). The '542 application is incorporated herein by reference.

It is noted that the operation described above in configuration with Fig. 18 assumes an ideal blocking diode 204. In practice, there will be a voltage drop across diode 204 in the forward direction and a leakage current will flow in the reverse direction. However, by properly choosing the characteristics of the diode 204, any adverse impact caused by these "real-life" parameters can be minimized or eliminated.

It is further noted that the blocking diode 204 could be eliminated, thereby simplifying the number of components involved. However, if diode 204 is removed, the sensing drive signal could potentially stimulate the heart. This should not happen, however, if the sensing drive signal is synchronized with the heart's refractory period, as it would be as described above. This is because the heart is not capable of being stimulated while it is refractory.

Fig. 19 provides a sensor process development tree of the assembly procedure. The main processes are described below.

The Fused Ring-Tube Assembly 205 includes fusing the glass tube and the two titanium end seal rings, wherein the seal produced must be hermetic, fitting well over the titanium sensor body and removing any oxides therefrom. Platinum-irridium feedthroughs and the titanium sensor body are fired to produce Titanium Bodies with Feedthroughs 206 wherein said feedthroughs must be hermetic, insulated, and able to withstand laser weld. The Complete Hybrid Assembly 207 comprises the hybrid substrate, one LED, two phototransistors, one resistor, and the reflector. The assembled hybrid must pass conventional hybrid lab quality control (Q.C.) requirements, laser weld, long-term aging, and shock under load. The Hybrid-Body Assembly 208 is formed by bonding the hybrid substrate to the titanium sensor body and welding the feedthroughs to the hybrid substrate. The mounted Hybrid-Body Assembly 208 must then pass laser weld, thermal aging, and shock. To provide a hermetic seal over the sensor, the Hybrid-Body Assembly 208 is slideably mounted into the Fused Ring-Tube Assembly 205 and laser welded to produce the Hermetic Sensor 209 such that the titanium-glass tube forms a hermetic seal with the titanium body with only minimal residual stress on the glass and no effect on the hybrid. Finally the proximal platinum-irridium feedthrough is crimped to proximal portion of the conductor. The other feedthrough is welded to the stylet tube on the distal side. Epoxy is potted between the protruding protions of the end rings. The resultant Sensor-Tube Assembly 210 must provide a reliable connection to the lead coils and insulation and pass a high potential (Hypot) dielectric test.

The LED 32 of the sensor 42, in the preferred embodiment, is a commercially available LED chip, type URDA-35E, commercially available from Shin-Itzu. The phototransistors 34 and 36 are likewise commercially available components, type F5OB, available from Siemens. Equivalent devices are available from numerous other manufacturers. As has been indicated, oxygen staturation of the blood is estimated by analyzing the optical intensity of reflected light at a specific wavelength (660 nm).

As thus described, a hermetically sealed sensor is disclosed that can advantageously be embedded within a pacing lead adapted for implantation within a human body. In order to sense the reflective properties of the blood to which the sensor is exposed, and in order to avoid interference with the normal operation of the pacemaker, the sensor can be activated during a time when the pacing lead is not being used by the pacemaker. These sensed reflective properties, in turn, provide an indication of the oxygen content of the blood. This information is then processed and used by a rate-responsive pacemaker in order to adjust the pacing interval of the pacemaker in an appropriate manner.

While the invention described herein has been described with reference to a particular embodiment and application thereof. For example, while the sensor has been described as being placed in one chamber of the heart, it could also be placed (for some purposes) in the other chamber of the heart, or in another location where it would be exposed to the fluid being monitored. Similarly, many variations are possible relative to how the sensor is mounted in a pacing lead, what type of pacing lead is used, if any, and how electrical contact is made with the sensor. Accordingly, the true scope of the invention should be determined with reference to the claims set forth below.

## Claims

1. A hermetically sealed, implantable medical sensor (42), preferably used with an implantable rate-responsive pacing system (56) to sense a desired parameter associated with body tissue, including body fluids, in which said medical sensor (42) is implanted, said sensor (42) comprising:
a hollow tube having sealed window means (142) therein;
a substrate (110) inserted into said tube;
light-emitting diode means (32) placed on said substrate (110) for emitting light of a prescribed frequency range in response to a drive signal, said light-emitting diode means (32) being positioned on said substrate (110) so as to permit at least a portion of said light to pass through said window means (142), contact body tissue outside of said window means (142), and reflect back through said window means;
phototransistor means (34, 36) placed on said substrate (110) for receiving light of a prescribed frequency range received through said window means (142), and for generating an output signal having characteristics that vary as a function of at least one parameter of the light received through said window means (142);
connection means (113, 114, 118, 120, 122, 123) for connecting said light-emitting diode means (32) and said phototransistor means (34, 36) in a desired circuit configuration, and for connecting a first terminal (112) to said light-emitting diode means (32), and for connecting a second terminal (116) to said phototransistor means (34, 36);
sealing means (145, 147, 101, 103) sealably secured to each end of said tube for sealing said substrate (110), light-emitting diode means (32), phototransistor means (34, 36), and connection means (113, 114, 118, 120, 122, 123) within said tube, said sealing means (145, 147, 101, 103) including feedthrough means (132, 134) for making electrical contact from a point external said tube with said first and second terminals (112, 116);
means for generating (46) said drive signal and applying it through said first and second terminals (112, 116) to said light-emitting diode means (32) said drive signal generating means (46) including means (72, 74, 78, 80) for generating a mono-phasic stair-stepped drive current that excites said light-emitting diode means (32) with a first current level for a first prescribed time period, and that excites said light-emitting diode means (32) with a second current level for a second time period immediately subsequent to said first time period; and
means (48) for monitoring through said first and second terminals (112, 116) the output signal generated by said phototransistor means (34, 36), and for processing said signal to provide a sensor signal indicative of said at least one parameter of the light received through said window means (142), said sensor signal thereby providing a measure of a desired characteristic of said body tissue as measured by the manner in which said at least one parameter of light is affected by coming in contact with said body tissue.

2. The medical sensor of claim 1 wherein said means for monitoring and processing (48) the output signal generated by said phototransistor means (34, 36) comprises means (90, 92, 94, 96) for determining the amount of change in the output signal as a result of the change in the amount of light reflected back into said window means (142) from said first time period to said second time period.

3. The medical sensor of claim 1 or 2, wherein said light-emitting diode means (32) emits light of a frequency that is affected by the oxygen content of blood.

4. The medical sensor of any of the claims 1 - 3, wherein said hollow tube is made from a material that is substantially transparent to the light emitted by said light-emitting diode means (32) and the light received by said phototransistor means (34, 36), said light transparent material thereby comprising the sealed window means (142) through which said light passes.

5. The medical sensor of claim 4 wherein said hollow tube material comprises soda lime glass.

6. The medical sensor of any of the claims 1 - 5 wherein said sealing means (101, 103, 145, 147) comprises a metal ring (144, 146) that is inserted into each end of said hollow tube, said metal rings (144, 146) having said feedthrough means (132, 134) sealably passing therethrough, said metal rings (144, 146) being sealably bonded to said hollow tube around the edges of said tube so as to form a tight seal fo at least 10⁻⁸ cc air/sec at 1,013·10⁵ Pa (one atmosphere pressure).

7. The medical sensor of claim 6 wherein said metal rings (144, 146) are made from titanium, and wherein the sealed bond between said metal rings (144, 146) and the edges of the hollow soda lime glass tube is a chemical bond formed between the titanium and the soda lime glass.

8. The medical sensor of any of the claims 1 - 7 wherein said light-emitting diode means (32) comprises a light-emitting diode chip (32) bonded to said substrate (110) substantially in the center thereof, and further including a reflector envelope (150) placed between said light-emitting diode chip (32) and a portion of the window means (142) of said hollow tube, whereby the light emitted by said light-emitting diode chip (32) is directed substantially upward and out through said window means (142).

9. The medical sensor of any of the claims 1 - 8 wherein said phototransistor means (34, 36) comprises at least two phototransistor chips (34, 36) that are bonded to said substrate (110), one phototransistor chip (34) being positioned on one side of said light-emitting diode means (32) but still under a portion of said window means (142); and the other phototransistor chip (36) being positioned on the other side of said light-emitting diode means (32), but still under another portion of said window means (142); said at least two phototransistor chips (34, 36) being electrically connected in parallel by said connection means (120, 122, 123, 118, 124, 126).

10. The medical sensor of any of the claims 1 - 9 wherein said sensor forms an integral part of an implantable stimulating lead (60) having a connector (62) at one end thereof and electrode means (66) at the other end thereof, the electrode means (66) comprising means (170) for electrically contacting body tissue when the lead (60) is implanted in a body, and the connector comprising a means for interfacing the lead (60), both electrically and physically, with said pacing system (56); a first insulated conductor (70, 182, 190) having a distal end coupled to the electrode means (66) and a proximal end coupled to the connector (62) and means (68, 180, 194, 200) for transmitting the drive signal and the output signal between the sensor means (42) and the connector (62).

11. The medical sensor of claim 10 further comprising a diode (204) and a second conductor (202); wherein said proximal end of said first conductor (200) is coupled to said distal end of said first conductor (200) through said diode (204); said proximal end is also electrically connected to said means (200) for transmitting said drive signal to said sensor; said second conductor (202) is electrically connected to a ring electrode (172) located near a distal end of said second conductor (202) and to said feedthrough means to said housing.

12. The medical sensor of claim 10 wherein said means for transmitting said drive signal to said sensor means comprises a second conductor coupled at a proximal end to said connector and coupled at a distal end to the feedthrough means of said housing.

13. The medical sensor of claim 12 wherein the feed-through means (132, 134) of said housing is further coupled to said first conductor.

14. The medical sensor of claim 12 or 13 wherein said lead (60) further includes a third conductor (192), electrically insulated from said first and second conductors (180, 182), said third conductor (192) being electrically connected to a ring electrode (172) located near a distal end of said lead (60).

15. The medical sensor of claim 14 wherein said third conductor passes through a poriton of said sensor means without making electrical contact therewith.

16. The medical sensor of claim 14 wherein said third conductor (198) meakes electrical contact with said sensor means.

17. The medical sensor of claim 14 wherein said first and third conductors (190, 192) are coaxial within said lead.

18. The medical sensor of claim 12 wherein said lead (60) comprises a bipolar lead having a ring electrode (172) and a tip electrode (170), and wherein said feed-through means of said housing includes first and second feedthrough terminals (132, 134), and further wherein one of said first and second conductors (180, 190) is electrically connected to said first feedthrough terminal (132), and the other (190) of said first and second conductors is electricallty connected to said second feedthrough terminal (134).

19. The medical sensor of claim 18 wherein said first and second conductors are coaxial within said lead.

20. The medical sensor of claim 12 wherein said lead (60) further includes a third conductor, electrically insulated from said first and second conductors, said third conductor being connected to the proximal end of said housing, and wherein the body of said housing is also used as the ring electrode.

21. A method of using a medical sensor that includes light-emitting diode means for emitting a light pulse that is directed to a desired type of organic material to be analyzed, which light pulse is then received by phototransistor means, a desired property of said organic material being determinable in accordance with the manner in which at least one detectable property of the light pulse is affected by the organic material; said method comprising the steps of:
(a) exciting said light-emitting diode means with a first stair-stepped drive current for a first period of time;
(b) exciting said light-emitting diode means with a second stair-stepped drive current of same polarity as the first drive current for a second period of time immediately subsequent said first period of time;
(c) monitoring the light pulses received by said phototransistor means during said first and second periods of time to determine the change in said light pulses as a result of having come in contact with the organic material; and
(d) processing the amount of change identified in step (c) to determine a desired property of the organic material.

22. The method of claim 21 wherein the second drive current leven used in step (b) to excite said light-emitting diode means is less than the first drive current level used in step (a).

23. The method of claim 21 or 22 wherein said organic material being analyzed by said medical sensor comprises blood, and further wherein the detectable property monitored by said medical sensor is the amount of the light pulse that is reflected from said blood, said reflected light amount being relatable by the processing of step (d) to the amount of oxygen in the blood.

## Patentansprüche

1. Hermetisch abgedichteter, implantierbarer, medizinischer Sensor (42), vorzugsweise zur Verwendung in Verbindung mit einem implantierbaren, auf die Herzfrequenz ansprechenden Schrittmachersystems (56), um einen mit dem Körpergewebe verbundenen, gewünschten Parameter, einschließlich Körperflüssigkeiten, zu erfassen, wobei der medizinische Sensor (42) implantiert wird, wobei der Sensor (42) umfaßt:
ein hohles Rohr mit versiegelten Fenstermitteln (142),
ein Substrat (110), das in das Rohr eingesetzt ist,
eine auf dem Substrat (110) angeordnete LED-Diodeneinrichtung (32), um Licht eines vorgeschriebenen Frequenzbereichs in Abhängigkeit von einem Antriebssignal abzugeben, wobei die LED-Diodeneinrichtung (32) auf dem Substrat (110) derart angeordnet ist, daß sie so gestattet, daß mindestens ein Teil des Lichts durch das Fenster (142) geht, das Körpergewebe außerhalb des Fensters (142) kontaktiert und durch das Fenster zurückreflektiert wird,
eine auf dem Substrat (110) angeordnete Phototransistoreinrichtung (34, 36) zum Empfangen von Licht eines vorgeschriebenen Frequenzbereichs, das durch das Fenster (142) hindurch aufgenommen wird und zum Erzeugen eines Ausgangssignal mit Eigenschaften, die in Abhängigkeit von mindestens einem Parameter des Lichts variieren, das durch das Fenster (142) hindurch empfangen wird,
eine Verbindungseinrichtung (113, 114, 118, 120, 122, 123) zum Verbinden der LED-Diodeneinrichtung (32) und der Phototransistoreinrichtung (34, 36) in einer gewünschten Schaltungskonfiguration und zum Verbinden eines ersten Anschlusses (112) mit der LED-Diodeneinrichtung (32) und zum Verbinden eines zweiten Anschlusses (116) mit der Phototransistoreinrichtung (34, 36),
eine Abdichteinrichtung (145, 147, 101, 103), die abdichtbar an jedem Ende des Rohrs befestigt ist, um das Substrat (110), die LED-Diodeneinrichtung (32), die Phototransistoreinrichtung (34, 36) und die Verbindungseinrichtung (113, 114, 118, 120, 122, 123) innerhalb des Rohrs abzudichten, wobei die Abdichteinrichtung (145, 147, 101, 103) Durchführeinrichtungen (132, 134) zum Herstellen eines elektrischen Kontakts von einem Punkt außerhalb des Rohrs mit den ersten und zweiten Anschlüssen (112, 116) aufweist,
eine Einrichtung (46) zum Erzeugen des Antriebssignals und dessen Anlegen durch die ersten und zweiten Anschlüsse (112, 116) an die LED-Diodeneinrichtung (32), wobei die Antriebssignalerzeugungseinrichtung (46) eine Einrichtung (72, 74, 78, 80) zum Erzeugen eines stufenförmigen Einphasenantriebsstroms enthält, der die LED-Diodeneinrichtung (32) mit einem ersten Strompegel während eines ersten vorgeschriebenen Zeitraums erregt und die die LED-Diodeneinrichtung (32) mit einem zweiten Strompegel während eines zweiten Zeitraums unmittelbar nach dem ersten Zeitraum erregt, und
eine Einrichtung (48), um durch die ersten und zweiten Anschlüsse (112, 116) das von der Phototransistoreinrichtung (34, 36) erzeugte Ausgangssignal zu überwachen und um das Signal zu verarbeiten, um ein Sensorsignal zu schaffen, daß den mindestens einen Parameter des durch das Fenster (142) hindurch empfangenen Lichts anzeigt, wodurch das Sensorsignal dadurch eine Größenordnung einer gewünschten Eigenschaft des Körpergewebes wie durch die Weise gemessen liefert, in der mindestens ein Parameter von Licht durch das Kontaktieren des Körpergewebes beeinflußt wird.

2. Medizinischer Sensor nach Anspruch 1, **dadurch gekennzeichnet, daß** die Einrichtung zur Überwachung und Verarbeitung (48) des von der Phototransistoreinrichtung (34, 36) erzeugten Ausgangssignals eine Einrichtung (90, 92, 94, 96) zur Bestimmung der Größe der Änderung des Ausgangssignals als Ergebnis der Änderung der Größe des in das Fenster (142) hinein reflektierten Lichts von dem ersten Zeitraum zu dem zweiten Zeitraum, umfaßt.

3. Medizinischer Sensor nach Anspruch 1 oder 2, **dadurch gekennzeichnet, daß** die LED-Diodeneinrichtung (32) Licht einer Frequenz abgibt, die durch den Sauerstoffgehalt des Bluts beeinflußt wird.

4. Medizinischer Sensor nach einem der Ansprüche 1 bis 3, **dadurch gekennzeichnet, daß** das hohle Rohr aus einem Material hergestellt ist, daß im wesentlichen dem von der LED-Diodeneinrichtung (32) abgegebenen Licht und dem von der Phototransistoreinrichtung (34, 36) empfangenen Licht gegenüber durchlässig ist, wobei das lichtdurchlässige Material dadurch das abgedichtete Fenster (142) umfaßt, durch welches das Licht geht.

5. Medizinischer Sensor nach Anspruch 4, **dadurch gekennzeichnet, daß** das Material des hohlen Rohrs Alkaliglas umfaßt.

6. Medizinischer Sensor nach einem der Ansprüche 1 bis 5, **dadurch gekennzeichnet, daß** die Abdichteinrichtung (101, 103, 145, 147) einen Metallring (144, 146) aufweist, der in jedes Ende des hohlen Rohrs eingesetzt ist, wobei die Metallringe (144, 146) diese Durchführeinrichtung (132, 134) aufweisen, die abdichtbar dort hindurch geführt ist, wobei die Metallringe (144, 146) abdichtbar mit dem hohlen Rohr um die Ränder des Rohrs herum verbunden sind, um eine dichte Abdichtung von mindestens 10⁻⁸ cm³ Luft/Sek. bei 1,013 . 10⁵ Pa (einem Atmosphärendruck) zu bilden.

7. Medizinischer Sensor nach Anspruch 6, **dadurch gekennzeichnet, daß** die Metallringe (144, 146) aus Titan hergestellt sind, und daß die abgedichtete Verbindung zwischen den Metallringen (144, 146) und den Rändern des hohlen Rohrs aus Alkaliglas eine zwischen dem Titan und dem Alkaliglas gebildete, chemische Bindung ist.

8. Medizinischer Sensor nach einem der Ansprüche 1 bis 7, **dadurch gekennzeichnet, daß** die LED-Diodeneinrichtung (32) einen LED-Diodenchip (32) umfaßt, der mit dem Substrat (110) im wesentlichen in dessen Mittelpunkt verbunden ist, und weiterhin eine Reflektorhülle (150) umfaßt, die zwischen dem LED-Diodenchip (32) und einem Bereich des Fensters (142) des hohlen Rohrs angeordnet ist, wodurch das von dem LED-Diodenchip (32) abgegebene Licht im wesentlichen nach oben und aus dem Fenster (142) hinaus gerichtet ist.

9. Medizinischer Sensor nach einem der Ansprüche 1 bis 8, **dadurch gekennzeichnet, daß** die Phototransistoreinrichtung (34, 36) mindestens zwei Phototransistorchips (34, 36) umfaßt, die mit dem Substrat (110) verbunden sind, wobei der eine Phototransistorchip (34) an einer Seite der LED-Diodeneinrichtung (32), aber noch unter einem Bereich des Fensters (142) angeordnet ist und der andere Phototransistorchip (36) an der anderen Seite der LED-Diodeneinrichtung (32), aber noch unter einem anderen Bereich des Fensters (142) angeordnet ist, wobei die mindestens zwei Phototransistorchips (34, 36) durch die Verbindungseinrichtung (120, 122, 123, 118, 124, 126) elektrisch parallel geschaltet sind.

10. Medizinischer Sensor nach einem der Ansprüche 1 bis 9, **dadurch gekennzeichnet, daß** der Sensor einen einstückigen Teil einer implantierbaren, stimulierenden Leitung (60) mit einem Verbinder (62) an deren einem Ende und Elektrodenmitteln (66) an deren anderem Ende besitzt, wobei die Elektrodenmittel (66) Mittel (170) zum elektrischen Kontaktieren von Körpergewebe besitzen, wenn die Leitung (60) in einen Körper implantiert ist und der Verbinder ein Mittel besitzt, um die Leitung (60) sowohl elektrisch als auch gegenständlich mit dem Schrittmachersystem (56) über eine Schnittstelle anzuschließen, wobei das distale Ende eines ersten isolierten Leiters (70, 182, 190) mit einem Elektrodenmittel (66) gekoppelt ist und dessen proximales Ende mit dem Verbinder (62) und Einrichtungen (68, 180, 194, 200) gekoppelt ist, um das Antriebssignal und das Ausgangssignal zwischen der Sensoreinrichtung (42) und dem Verbinder (62) zu übertragen.

11. Medizinischer Sensor nach Anspruch 10, der weiterhin eine Diode (204) und einen zweiten Leiter (202) umfaßt, **dadurch gekennzeichnet, daß** das proximale Ende des ersten Leiters (200) an dem distalen Ende des ersten Leiters (200) über die Diode (204) gekoppelt ist, das proximale Ende auch mit der Einrichtung (200) zum Übertragen des Antriebssignals an den Sensor elektrisch verbunden ist, der zweite Leiter (202) elektrisch mit einer Ringelektrode (172), die sich in der Nähe des distalen Endes des zweiten Leiters (202) befindet, und mit der Durchführeinrichtung zu dem Gehäuse verbunden ist.

12. Medizinischer Sensor nach Anspruch 10, **dadurch gekennzeichnet, daß** die Einrichtung zum Übertragen des Antriebssignals an den Sensor einen zweiten Leiter aufweist, der mit dem proximalen Ende des Verbinders gekoppelt ist und an dem distalen Ende mit der Durchführeinrichtung des Gehäuses gekoppelt ist.

13. Medizinischer Sensor nach Anspruch 12, **dadurch gekennzeichnet, daß** die Durchführeinrichtung (132, 134) des Gehäuses weiterhin mit dem ersten Leiter verbunden ist.

14. Medizinischer Sensor nach Anspruch 12 oder 13, **dadurch gekennzeichnet, daß** die Leitung (60) weiterhin einen dritten Leiter (192) umfaßt, der gegenüber dem ersten und dem zweiten Leiter (180, 182) elektrisch isoliert ist, wobei der dritte Leiter (192) elektrisch mit einer Ringelektrode (172) verbunden ist, die sich nahe dem distalen Ende der Leitung (60) befindet.

15. Medizinischer Sensor nach Anspruch 14, **dadurch gekennzeichnet, daß** der dritte Leiter durch einen Bereich des Sensors hindurchgeht, ohne einen elektrischen Kontakt damit herzustellen.

16. Medizinischer Sensor nach Anspruch 14, **dadurch gekennzeichnet, daß** der dritte Leiter (198) einen elektrischen Kontakt mit dem Sensor herstellt.

17. Medizinischer Sensor nach Anspruch 14, **dadurch gekennzeichnet, daß** der erste und der dritte Leiter (190, 192) koaxial innerhalb der Leitung liegen.

18. Medizinischer Sensor nach Anspruch 12, **dadurch gekennzeichnet, daß** die Leitung (60) eine bipolare Leitung mit einer Ringelektrode (172) und einer Spitzenelektrode (170) aufweist, und die Durchführeinrichtung des Gehäuses erste und zweite Durchführanschlüsse (132, 134) besitzt und einer der ersten und zweiten Leiter (180, 190) elektrisch mit dem ersten Durchführanschluß (132) verbunden ist und der andere (190) der ersten und zweiten Leiter elektrisch mit dem zweiten Durchführanschluß (134) verbunden ist.

19. Medizinischer Sensor nach Anspruch 18, **dadurch gekennzeichnet, daß** die ersten und zweiten Leiter innerhalb der Leitung koaxial liegen.

20. Medizinischer Sensor nach Anspruch 12, **dadurch gekennzeichnet, daß** die Leitung (60) weiterhin einen dritten Leiter umfaßt, der elektrisch gegenüber dem ersten und dem zweiten Leiter isoliert ist, wobei der dritte Leiter mit dem proximalen Ende des Gehäuses verbunden ist und der Körper des Gehäuses auch als Ringelektrode verwendet wird.

21. Verfahren zur Verwendung eines medizinisches Sensors, welcher eine LED-Diodeneinrichtung zum Abgeben eines Lichtimpulses umfaßt, der auf einen gewünschten Typ von zu analysierendem, organischen Material gerichtet ist, wobei der Lichtimpuls dann durch eine Phototransistoreinrichtung empfangen wird, wobei eine gewünschte Eigenschaft des organischen Materials in Übereinstimmung mit der Weise bestimmbar ist, in der mindestens eine erfaßbare Eigenschaft des Lichtimpulses durch das organische Material beeinflußt wird, wobei das Verfahren folgende Schritte umfaßt:
a) Erregen der LED-Diodeneinrichtung mit einem ersten stufenförmigen Antriebsstrom während eines ersten Zeitraums,
b) Erregen der LED-Diodeneinrichtung mit einem zweiten stufenförmigen Antriebsstrom der gleichen Polarität wie der erste Antriebsstrom während eines zweiten Zeitraums unmittelbar nach dem ersten Zeitraum,
c) Überwachen der von der Phototransistoreinrichtung während des ersten und des zweiten Zeitraums empfangenen Lichtimpulses, um die Änderung der Lichtimpulse als Folge des Kontaktierens des organischen Materials zu bestimmen, und
d) Verarbeiten der Größe der in Schritt (c) identifizierten Änderung, um eine gewünschte Eigenschaft des organischen Materials zu bestimmen.

22. Medizinischer Sensor nach Anspruch 21, **dadurch gekennzeichnet, daß** der in Schritt (b) zur Erregung der LED-Diodeneinrichtung verwendete, zweite Antriebsstrompegel geringer ist als der erste in Schritt (a) verwendete Antriebsstrompegel.

23. Verfahren nach Anspruch 21 oder 22, **dadurch gekennzeichnet, daß** das organische Material, das durch den medizinischen Sensor analysiert wird, Blut ist, und die von dem medizinischen Sensor überwachte, erfaßbare Eigenschaft die Größe des Lichtimpulses ist, die von dem Blut reflektiert wird, wobei die Menge des reflektierten Lichts durch die Verarbeitung von Schritt (d) in Beziehung zu dem Sauerstoffgehalt im Blut gesetzt werden kann.

## Revendications

1. Capteur (42) médical implantable, fermé de manière étanche, utilisé de préférence en association avec un système (56) de stimulation implantable fonctionnant en réponse à une fréquence afin de détecter un paramètre souhaité associé au tissu corporel, y compris les fluides corporels, dans lequel le capteur (42) médical est implanté, ce capteur (42) comprenant :
un tube creux ayant une fenêtre (142) fermée de manière étanche en son sein ;
un substrat (110) inséré dans le tube ;
des moyens (32) formant diodes émettrices de lumière disposées sur le substrat (110) afin d'émettre de la lumière dont la fréquence est comprise dans une gamme prescrite en réponse à un signal d'attaque, ces moyens (32) formant diodes émettrices de lumière étant placées sur le substrat (110) de façon à permettre qu'au moins une partie de la lumière passe par la fenêtre (142), vienne en contact avec le tissu corporel à l'extérieur de la fenêtre (142), et soit réfléchie dans la fenêtre ;
des moyens (34, 36) à phototransistors placés sur le substrat (110) afin de recevoir de la lumière ayant une fréquence comprise dans une gamme prescrite et qui est reçue par la fenêtre (142), et de produire un signal de sortie ayant des caractéristiques qui varient en fonction d'au moins un paramètre de la lumière reçue par la fenêtre (142);
des moyens (113, 114, 118, 120, 122, 123) de connexion destinés à connecter les moyens (32) formant diodes émettrices de lumière et les moyens (34, 36) à phototransistors selon une configuration de circuit souhaitée, et destinés à connecter une première borne (112) aux moyens (32) formant diodes émettrices de lumière et destinés à connecter une seconde borne (116) aux moyens (34, 36) à phototransistors ;
des moyens (145, 147, 101, 103) d'étanchéité fixés de manière étanche à chaque extrémité du tube afin de rendre étanche le substrat (110), les moyens (32) formant diodes émettrices de lumière, les moyens (34, 36) phototransistor et les moyens (113, 114, 118, 120, 122, 123) de connexion à l'intérieur du tube, ces moyens d'étanchéité (145, 147, 101, 103) comportant des moyens (132, 134) de traversée destinés à réaliser un contact électrique entre un point extérieur du tube et les première et seconde (112, 116) bornes ;
des moyens (46) destinés à produire le signal d'attaque et à l'appliquer aux moyens (32) formant diodes émettrices de lumière par l'intermédiaire des première et seconde (112, 116) bornes, ces moyens (46) de production du signal d'attaque comportant des moyens (72, 74, 78, 80) destinés à produire un courant d'attaque monophasé en forme d'échelons qui excite les moyens (32) formant diodes émettrices de lumière avec un premier niveau de courant pendant une première durée prescrite, et qui excite les moyens (32) formant diodes émettrices de lumière avec un second niveau de courant pendant une seconde durée qui suit immédiatement la première durée ; et
des moyens (48) pour contrôler le signal de sortie des première et seconde (112, 116) bornes produit par les moyens (34, 36) à phototransistor et pour traiter ce signal afin de procurer un signal de détection qui représente une indication dudit au moins un paramètre de la lumière reçue par la fenêtre (142), ce signal de détection procurant ainsi une mesure d'une caractéristique souhaitée du tissu corporel tel que mesuré par la manière dont ledit au moins un paramètre de lumière est affecté par le fait de venir en contact avec le tissu corporel.

2. Capteur médical selon la revendication 1, dans lequel les moyens (48) de contrôle et de traitement du signal de sortie produit par les moyens (34, 36) à phototransistor comprennent des moyens (90, 92, 94, 96) destinés à déterminer la variation du signal de sortie sous l'effet d'une variation de la quantité de lumière réfléchie dans la fenêtre (142) entre la première durée et la seconde durée.

3. Capteur médical selon la revendication 1 ou 2, dans lequel les moyens (32) formant diodes émettrices de lumière émettent de la lumière à une fréquence qui varie en fonction de la teneur en oxygène du sang.

4. Capteur médical selon l'une quelconque des revendications 1 à 3, dans lequel le tube creux est en un matériau qui est sensiblement transparent à la lumière émise par les moyens (32) formant diodes émettrices de lumière et à la lumière reçue par les moyens (34-36) à phototransistor, ce matériau transparent à la lumière comportant la fenêtre (142) fermée de manière étanche par laquelle la lumière passe.

5. Capteur médical selon la revendication 4, dans lequel le matériau du tube creux comprend du verre à base de soude et de chaux.

6. Capteur médical selon l'une quelconque des revendications 1 à 5, dans lequel les moyens (101, 103, 145, 147) d'étanchéité comportent un anneau (144, 146) métallique qui est inséré dans chaque extrémité du tube creux, les moyens (132, 134) de traversée passant de manière étanche dans ces anneaux (144, 146) métalliques, ces anneaux (144, 146) métalliques étant fixés de manière étanche au tube creux sur les bords du tube de manière à former un joint étanche, correspondant à au moins 10⁻⁸ cm³ d'air par seconde à la pression atmosphérique (1,013 10⁵ Pa)

7. Capteur médical selon la revendication 6 dans lequel les anneaux (144, 146) métalliques sont en titane, et dans lequel la fixation étanche entre les anneaux (144, 146) métalliques et les bords du tube creux en verre à base de soude et de chaux est une liaison chimique formée entre le titane et le verre à base de soude et de chaux.

8. Capteur médical selon l'une quelconque des revendications 1 à 7, dans lequel les moyens (32) formant diodes émettrices de lumière comprennent une puce (32) formant diode émettrice de lumière fixée au substrat (110) sensiblement en son centre, et comportant en outre une enveloppe (150) réfléchissante placée entre la puce (32) formant diode émettrice de lumière et une partie de la fenêtre (142) du tube creux, de sorte que la lumière émise par la puce (32) formant diode émettrice de lumière est dirigée sensiblement vers le haut en sortant par la fenêtre (142).

9. Capteur médical selon l'une quelconque des revendications 1 à 8 dans lequel les moyens (34, 36) à phototransistor comprennent au moins deux puces (34, 36) à phototransistor qui sont fixées au substrat (110), une puce (34) à phototransistor étant placée d'un côté des moyens (32) formant diodes émettrices de lumière mais encore sous une partie de la fenêtre (142) ; et l'autre puce (36) à phototransistor étant placée de l'autre côté des moyens (32) formant diodes émettrices de lumière, mais cependant encore sous une autre partie de la fenêtre (142), les dites au moins deux puces (34, 36) à phototransistor étant connectées électriquement en parallèle au moyen de moyens (120, 122, 123, 118, 124, 126) de connexion.

10. Capteur médical selon l'une quelconque des revendications 1 à 9, dans lequel le capteur est d'une pièce avec une ligne (60) de stimulation implanté ayant un connecteur (62) à l'une ses extrémités et des moyens (66) formant électrodes à son autre extrémité, les moyens (66) formant électrodes comprenant des moyens (170) pour venir en contact électrique avec le tissu corporel lorsque la ligne (60) est implantée dans un corps, et le connecteur comprend des moyens destinés à mettre en interface, à la fois électriquement et physiquement, la ligne (60) avec le système (56) de stimulation ; un premier conducteur (70, 182, 190) isolé ayant une extrémité distale couplée au moyen (66) formant électrode et une extrémité proximale couplée au connecteur (62) et des moyens (66, 180, 194, 200) destinés à transmettre le signal d'attaque et le signal de sortie entre les moyens (42) formant capteur et le connecteur (62).

11. Capteur médical selon la revendication 10 comprenant en outre une diode (204) et un second conducteur (202) ; dans lequel l'extrémité proximale du premier conducteur 200 est couplée à l'extrémité distale du premier conducteur (200) par l'intermédiaire de la diode (204) ; l'extrémité proximale est également connectée électriquement au moyen (200) afin de transmettre le signal d'attaque au capteur ; le second conducteur (202) est électriquement connecté à une électrode (172) à anneaux située à proximité d'une extrémité distale du second conducteur (202) ainsi qu'aux moyens de traversée du boîtier.

12. Capteur médical selon la revendication 10 dans lequel les moyens destinés à transmettre le signal d'attaque aux capteurs comprennent un deuxième conducteur couplé à une extrémité proximale du connecteur et couplé à une extrémité distale des moyens de traversée du boîtier.

13. Capteur médical selon la revendication 12 dans lequel les moyens (132, 134) de traversée du boîtier sont en outre couplés au premier conducteur.

14. Capteur médical selon la revendication 12 ou 13, dans lequel la ligne (60) comporte en outre un troisième conducteur (192), électriquement isolé des premier et deuxième conducteurs (180, 182), ce troisième conducteur (192) étant connecté électriquement à une électrode (172) en anneau située à proximité d'une extrémité distale de la ligne (60).

15. Capteur médical selon la revendication 14, dans lequel le troisième conducteur passe par une partie du capteur sans venir en contact électrique avec celui-ci.

16. Capteur médical selon la revendication 14, dans lequel le troisième conducteur (198) vient en contact électrique avec le capteur.

17. Capteur médical selon la revendication 14 dans lequel les premier et troisième conducteurs (190, 192) sont coaxiaux à l'intérieur de la ligne.

18. Capteur médical selon la revendication 12, dans lequel la ligne (60) comporte une ligne bipolaire ayant une électrode (172) en anneau et une électrode (170) en pointe, et dans lequel les moyens de traversée du boîtier comportent des première et seconde bornes (132, 134) de traversée, et dans lequel en outre l'un des deux premier et deuxième conducteurs (180,190) est connecté électriquement à la première borne (132) de traversée, et l'autre (190) des premier et deuxième conducteurs est connecté électriquement à la seconde borne (134) de traversée.

19. Capteur médical selon la revendication 18 dans lequel les premier et deuxième conducteurs sont coaxiaux à l'intérieur de la ligne.

20. Capteur médical selon la revendication 12 dans lequel la ligne (60) comporte en outre un troisième conducteur, isolé électriquement des premier et deuxième conducteurs, le troisième conducteur étant connecté à l'extrémité proximale du boîtier, et dans lequel le corps du boitier est également utilisé en tant qu'électrode en anneau.

21. Procédé d'utilisation d'un capteur médical qui comporte des moyens formant diodes émettrices de lumière destinés à émettre une impulsion lumineuse qui est dirigée vers un type souhaité de matériau organique à analyser, cette impulsion lumineuse étant ensuite reçue par des moyens à phototransistor, une propriété souhaitée du matériau organique pouvant être déterminée par la manière dont au moins une propriété détectable de l'impulsion lumineuse est affectée par le matériau organique ; ce procédé comprenant les étapes qui consistent à :
a) exciter les moyens formant diodes émettrices de lumière par un premier courant d'attaque en forme d'échelon pendant une première durée ;
b) exciter les moyens formant diodes émettrices de lumière par un second courant d'attaque en forme d'échelon de même polarité que le premier courant d'attaque pendant une seconde durée suivant immédiatement la première durée ;
c) contrôler les impulsions lumineuses reçues par les moyens à phototransistor pendant les première et seconde durée pour déterminer le changement des impulsions lumineuses à la suite du fait qu'elles sont venues en contact avec le matériau organique ; et
d) traiter la variation mise en évidence à l'étape (c) pour déterminer une propriété souhaitée du matériau organique.

22. Procédé selon la revendication 21, dans lequel le deuxième courant d'attaque utilisé à l'étape (b) pour exciter les moyens formant diodes émettrices de lumière est inférieur au premier courant d'attaque utilisé à l'étape (a).

23. Procédé selon la revendication 21 ou 22 dans lequel le matériau organique analysé par le capteur médical comprend du sang et dans lequel en outre la propriété détectable contrôlée par le capteur médical est la quantité de l'impulsion lumineuse qui est réfléchie par le sang, cette quantité de lumière réfléchie pouvant être mise en relation avec la quantité d'oxygène dans le sang au moyen du traitement de l'étape (d).
